# EUROPEAN PATENT SPECIFICATION

(11) **EP 0 729 351 B1**
(45) Date of publication and mention of the grant of the patent: **13.09.2000**
(21) Application number: 95901208.9
(22) Date of filing: 10.11.1994
(51) Int. Cl.: A61K 9/127

(54) **VESICLES WITH CONTROLLED RELEASE OF ACTIVES**
VESIKEL MIT GESTEUERTER WIRKSTOFFFREISETZUNG
VESICULES A TAUX CONTROLE DE LIBERATION DES PRINCIPES ACTIFS

(30) Priority: 16.11.1993 US 153657
(43) Date of publication of application: 04.09.1996
(73) Proprietor: SkyePharma Inc., San Diego, CA 92121 (US)
(72) Inventor: SANKARAM, Mantripragada, San Diego, CA 92130 (US); KIM, Sinil, Solana Beach, CA 92705 (US)
(74) Representative: Hale, Peter
(86) International application number: PCT/US94/12957
(87) International publication number: WO 95/13796

(56) References cited:
- EP-A- 0 280 503
- EP-A- 0 315 467
- WO-A-88/04573
- US-A- 4 224 179
- US-A- 4 310 506
- US-A- 4 588 578
- US-A- 4 752 425
- US-A- 4 920 016
- US-A- 5 211 955
- CANCER RESEARCH 53, 1 April 1993, (KIM), "Prolongation of Drug Exposure in Cerebrospinal Fluid by Encapsulation into DepoFoam", pp. 1596-1598.

## Description

### BACKGROUND OF THE INVENTION

### 1. Field of the Invention

The invention relates to synthetic membrane vesicles and to compositions comprising them which are useful as a drug delivery system, to processes for their manufacture as well as to target delivery systems comprise these compositions.

### 2. Background of the Invention

Multivesicular liposomes are one of the three main types of liposomes, first made by Kim, *et al. (Biochim, Biophys. Acta*, 782:339-348, 1983), and are uniquely different from other lipid-based drug delivery systems such as unilamellar (Huang, *Biochemistry*, 8:334-352, 1969; Kim, *et al*., *Biochim. Biophys. Acta*, 646:1-10, 1981) and multilamellar (Bangham*, et al., J. Mol. Bio.*, 13:238-252, 1965) liposomes. In contrast to unilamellar liposomes, multivesicular particles contain multiple aqueous chambers per particle. In contrast to multilamellar liposomes, the multiple aqueous chambers in multivesicular particles are non-concentric.

The prior art describes a number of techniques for producing unilamellar and multilamellar liposomes; for example, U.S. Patent No. 4,522,803 to Lenk; 4,310,506 to Baldeschwieler; 4,235,871 to Papahadjopoulos; 4,224,179 to Schneider; 4,078,052 to Papahadjopoulos; 4,394,372 to Taylor; 4,308,166 to Marchetti; 4,485,054 to Mezei; and 4,508,703 to Redziniak. The prior art also describes methods for producing multivesicular liposomes that proved unstable in biological fluids (Kim, *et al., Biochim. Biophys. Acta,* 728:339-348, 1983). For a comprehensive review of various methods of unilamellar and multilamellar liposome preparation, refer to Szoka, *et al., Ann. Rev. Biophys. Bioeng.,*9:465-508, 1980.

In the method of Kim, *et al.* (*Biochim. Biophys. Acta,* 728:339-348, 1983), the encapsulation efficiency of small molecules, such as cytosine arabinoside, was low, and had a rapid release rate in biological fluids. Subsequent studies (Kim, *et al., Cancer Treat. Rep.,* 71:705-711, 1987) showed that the rapid release rate of encapsulated molecules in biological fluids can be improved by encapsulation in the presence of a hydrochloride.

Optimal treatment with many drugs requires maintenance of a drug level for a prolonged period of time. For example, optimal anti-cancer treatment with cell cycle-specific antimetabolites requires maintenance of a cytotoxic drug level for a prolonged period of time. Cytarabine is a highly schedule-dependent anti-cancer drug. Because this drug kills cells only when they are replicating DNA, a prolonged exposure at therapeutic concentration of the drug is required for optimal cell kill. Unfortunately, the half-life of cytarabine after an intravenous (IV) or subcutaneous (SC) dose is very short, with the half-life in the range of a few hours. To achieve optimal cancer cell kill with a cell cycle phase-specific drag like cytarabine, two major requirements need to be met: first, the cancer must be exposed to a high concentration of the drug without doing irreversible harm to the host; and second, the tumor must be exposed for a prolonged period of time so that all or most of the cancer cells have attempted to synthesize DNA in the presence of cytarabine.

Heretofore, control of the release rate was inflexible, and the choice of release-rate modifying agents was limited primarily to hydrohalides. For a drug-delivery system, it is highly advantageous to be flexible in controlling the release rate for encapsulated substances and to have a wide choice of release-rate modifying agents.

Accordingly, it is an object of the present invention to provide a slow-release depot preparation which provides a prolonged and sustained exposure of a biologically active substance at a therapeutic concentration, with a controlled release rate.

It is a further object of the present invention to provide a method of preparing such depot preparations.

Other and further objects, features, and advantages of the invention are inherent therein and appear throughout the specification and claims.

Slowing down the release rate of pharmaceutical substances from liposomes is known from EP-A- 0 280 503.

### SUMMARY OF THE INVENTION

The compositions of the present invention comprise synthetic membrane vesicles, i.e. lipid vesicles with multiple internal aqueous chambers formed by non-concentric layers and wherein the chambers contain one or more non hydrohalide release-rate modifying agents effective in slowing the release rate of the encapsulated biologically active substances. The present invention also provides methods of making such compositions.

The invention also relates to the use of a biologically active compound encapsulated in the above vesicles for the preparation of a pharmaceutical composition.

The present synthetic membrane vesicle compositions have high encapsulation efficiency, controlled release rate of the encapsulated substance, well defined, reproducible size distribution, spherical shape, adjustable average size that can be easily increased or decreased, and adjustable internal chamber size and number.

The process for producing these compositions comprises (1) mixing one or more volatile organic solvents and a lipid component containing at least one neutral lipid and at least one amphipathic lipid having one or more net negative charges; (2) adding into the organic solvent an immiscible first aqueous component containing one or more biologically active substances to be encapsulated; (3) adding to either or both the organic solvent and the first aqueous component, a release-rate modifying agent effective in slowing the release rate of the encapsulated biologically active substances; (4) forming a water-in-oil emulsion from the two immiscible components; (5) immersing the water-in-oil emulsion into a second immiscible aqueous component; (6) dispersing the water-in-oil emulsion to form solvent spherules containing in them multiple droplets of the first aqueous component; and (7) removing the organic solvents, such as by evaporation, from the solvent spherules to form the synthetic membrane vesicles. Addition of one or more release-rate modifying agents effective in slowing the release rate of the encapsulated biologically active substances in biological fluids and *in vivo* is essential.

The invention also pertains to a process for producing synthetic membrane vesicles comprising the steps of:
(a) forming a water-in-oil emulsion from two immiscible components containing at least one organic solvent, water, at least one biologically active substance, and at least one non-hydrohalide release-rate modifying agent;
(b) dispersing the water-in-oil emulsion into an aqueous component to form solvent spherules; and
(c) removing the organic solvent from the solvent spherules to form the synthetic membrane vesicles containing aqueous droplets with the biologically active substance and the release rate modifying agent dissolved therein..

### A BRIEF DESCRIPTION OF THE DRAWING

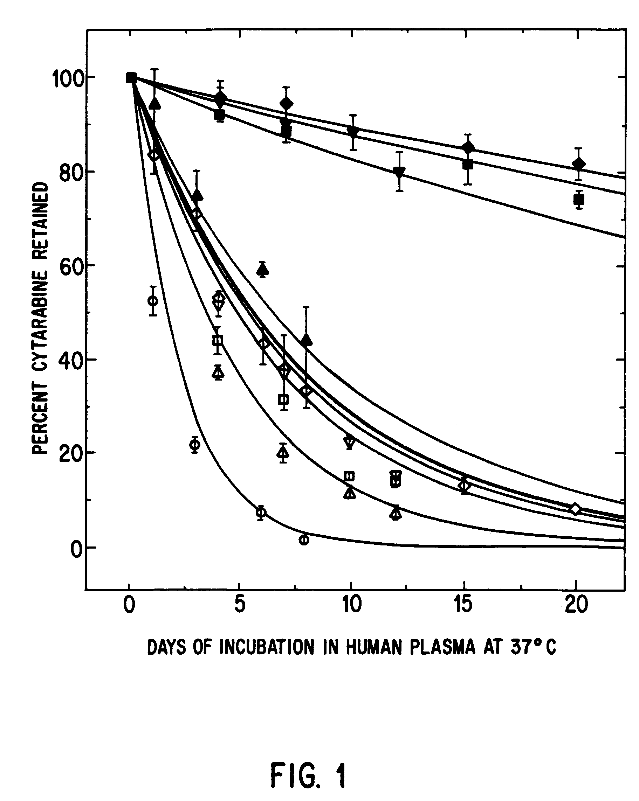

Figure 1 is a graph showing the rate of release of a drug from synthetic membrane vesicles suspended in human plasma at 37°C. The symbols used indicate the release rate modifying agent employed and are identified in Table 2.

### DESCRIPTION OF THE PREFERRED EMBODIMENT

The tent "synthetic membrane vesicles" as used throughout the specification and claims means man-made, microscopic lipid-vesicles consisting of lipid bilayer membranes, enclosing multiple non-concentric aqueous chambers. In contrast, unilamellar liposomes have a single aqueous chamber; and multilamellar liposomes have multiple "onion-skin" type of concentric membranes, in between which are shell-like concentric aqueous compartments.

The term "solvent spherule" as used throughout the specification and claims means a microscopic spheroid droplet of organic solvent, within which is multiple smaller droplets of aqueous solution. The solvent spherules are suspended and totally immersed in a second aqueous solution.

The term "neutral lipid" means oil or fats that have no membrane-forming capability by themselves and lack a hydrophilic "head" group.

The term "amphipathic lipids" means those molecules that have a hydrophilic "head" group and hydrophobic "tail" group and have membrane-forming capability.

The term "release-rate modifying agent" means molecules other than hydrohalides added during the process of making or manufacturing the synthetic membrane vesicles that are effective in either slowing or increasing the release rate of the encapsulated biologically active substances from the synthetic membrane vesicles.

Briefly, the method of the invention comprises making a "water-in-oil" emulsion by (1) dissolving amphipathic lipids in one or more volatile organic solvents for the lipid component, (2) adding to the lipid component an immiscible first aqueous component and a biologically active substance to be encapsulated, and (3) adding to either or both the organic solvent and the first aqueous component, a release-rate modifying agent effective in slowing the release rate of the encapsulated biologically active substances from the synthetic membrane vesicles, and then emulsifying the mixture mechanically.

In the emulsion, the water droplets suspended in the organic solvent will form the internal aqueous chambers, and the monolayer of amphipathic lipids lining the aqueous chambers will become one leaflet of the bilayer membrane in the final product. The emulsion is then immersed in a second aqueous component containing one or more nonionic osmotic agents and an acid-neutralizing agent of low ionic strength, such as a proton acceptor, preferably selected from free-base lysine, free-base histidine, or a combination thereof. Then the emulsion is agitated either mechanically, by ultrasonic energy, nozzle atomizations, and the like, or by combinations thereof, to form solvent spherules suspended in the second aqueous component.

The solvent spherules contain multiple aqueous droplets with the substance to be encapsulated dissolved in them. The organic solvent is removed from the spherules, preferably by evaporation of a volatile solvent, for instance by passing a stream of gas over the suspension. When the solvent is completely removed, the spherules convert into synthetic membrane vesicles. Representative gases satisfactory for use in evaporating the solvent include nitrogen, helium, argon, oxygen, hydrogen, and carbon dioxide.

The release-rate modifying agent is any molecule that is effective in slowing the rate of release of the encapsulated biologically active substances from the synthetic membrane vesicles in biological fluids and *in vivo*, with the result that the release rate of the substances is slower than that from synthetic membrane vesicles produced in the absence of such a release-rate modifying agent. The release-rate modifying agents include, but are not limited to, perchloric acid, nitric acid, formic acid, acetic acid, trifluoroacetic acid, trichloroacetic acid, sulfuric acid, phosphoric acid, and combinations thereof. The amounts of the release-rate modifying agents used are effective to provide a prolonged, sustained, and controlled rate of release at therapeutic levels of the encapsulated biologically active substances. For example, the concentration of the release-rate modifying agent in the organic solvent or the first aqueous component to which it is added is in the range from about 0.1 mM to about 0.5 M and preferably from about 10 mM to about 200 mM.

Many different types of volatile hydrophobic solvents such as ethers, hydrocarbons, halogenated hydrocarbons, or Freons may be used as the lipid-phase solvent. For example, diethyl ether, isopropyl and other ethers, chloroform, tetrahydrofuran, halogenated ethers, esters and combinations thereof are satisfactory.

In order to prevent the solvent spherules from sticking to each other and to the vessel wall, it is preferred that at least 1 percent molar ratio of an amphipathic lipid with a net negative charge be included in the spherules, that the suspending second aqueous solution have a very low ionic strength, and, when an acid is used, that an agent for neutralizing the acid be added to the second aqueous solution to form a concentration of from about 0.1 mM to about 0.5 M therein to prevent coalescence of the solvent spherules to form a messy scum. In addition, one or more nonionic osmotic agents, such as trehalose, glucose, or sucrose, may optionally be used in the suspending aqueous solution to keep the osmotic pressure within and without the membrane vesicles balanced.

Various types of lipids can be used to make the synthetic membrane vesicles, and the only two requirements are that one amphipathic lipid with a net negative charge and a neutral lipid be included. Examples of neutral lipids are triolein, trioctanoin, vegetable oil such as soybean oil, lard, beef fat, tocopherol, and combinations thereof. Examples of amphipathic lipids with net negative charges are cardiolipin, the phosphatidylserines, phosphatidyiglycerols, and phosphatidic acids.

The second aqueous component is an aqueous solution containing low ionic strength solutes such as carbohydrates including glucose, sucrose, lactose, and amino acids such as lysine, free-base histidine and combinations thereof.

Many and varied biological substances and therapeutic agents can be incorporated by encapsulation within the synthetic membrane vesicles.

The term "therapeutic agent" as used herein for the compositions of the invention includes, without limitation, drugs, radioisotopes, and immunomodulators. Similar substances are known or can be readily ascertained by one of skill in the art. There may be certain combinations of therapeutic agent with a given type of synthetic membrane vesicles that are more compatible than others. For example, the method for producing the synthetic membrane vesicles may not be compatible with the continued biological activity of a proteinaceous therapeutic agent. However, since conditions that would produce an incompatible pairing of a particular therapeutic agent with a particular dispersion system are well known, or easily ascertained, it is a matter of routine to avoid such potential problems.

The drugs that can be incorporated into the dispersion system as therapeutic agents include non-proteinaceous as well as proteinaceous drugs. The term "non-proteinaceous drugs" encompasses compounds that are classically referred to as drugs, such as mitomycin C, daunorubicin, vintlastine, AZT, and hormones. Of particular interest are anti-tumor cell-cycle specific drugs such as cytarabine, methotrexate, 5-fluorouracil (5-FU), floxuridine (FUDR), bleomycin, 6-mercaptopurine, 6-thioguanine, fludarabine phosphate, vincristine, and vinblastine.

Examples of proteinaceous materials that can be incorporated into the synthetic membrane vesicles are DNA, RNA, proteins of various types, protein hormones produced by recombinant DNA technology effective in humans, hematopoietic growth factors, monokines, lymphokines, tumor necrosis factor, inhibin, tumor growth factor alpha and beta, Mullerian inhibitory substance, nerve growth factor, fibroblast growth factor, platelet-derived growth factor, pituitary and hypophyseal hormones including LH and other releasing hormones, calcitonin, proteins that serve as immunogens for vaccination, and DNA and RNA sequences.

The following TABLE 1 includes a list of representative biologically active substances effective in humans that can be encapsulated in synthetic membrane vesicles in the presence of a release-rate modifying agent of the invention, and also includes biologically active substances effective for agricultural uses.

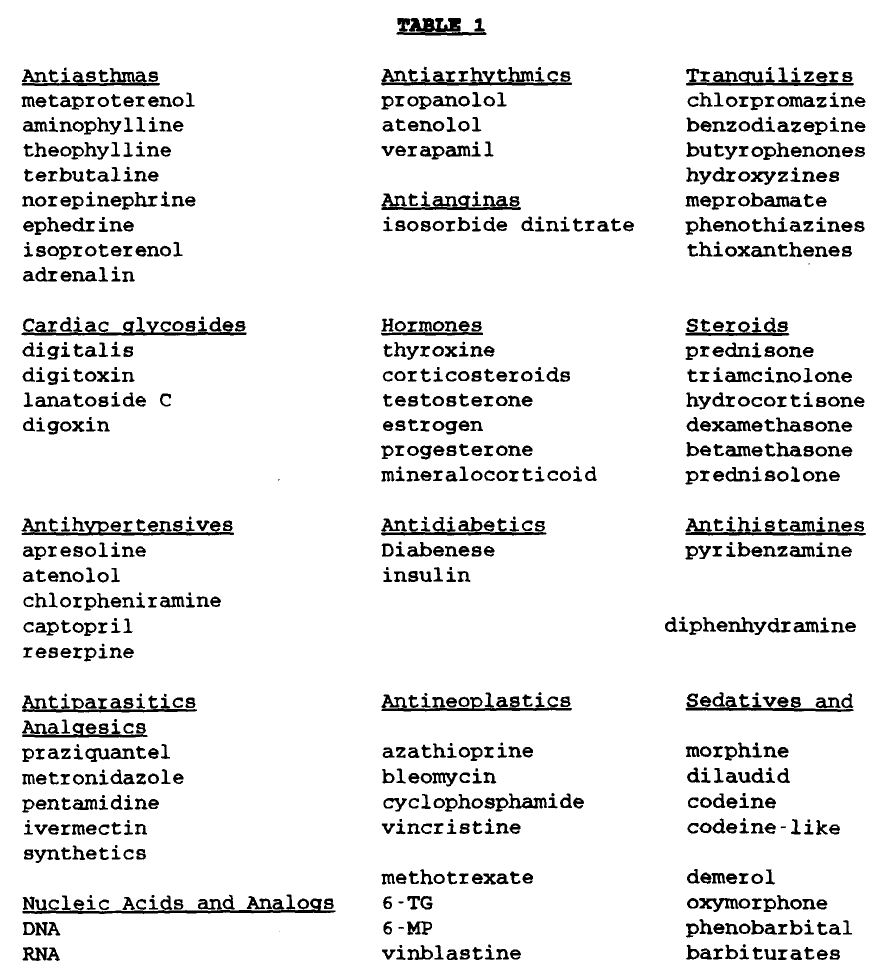

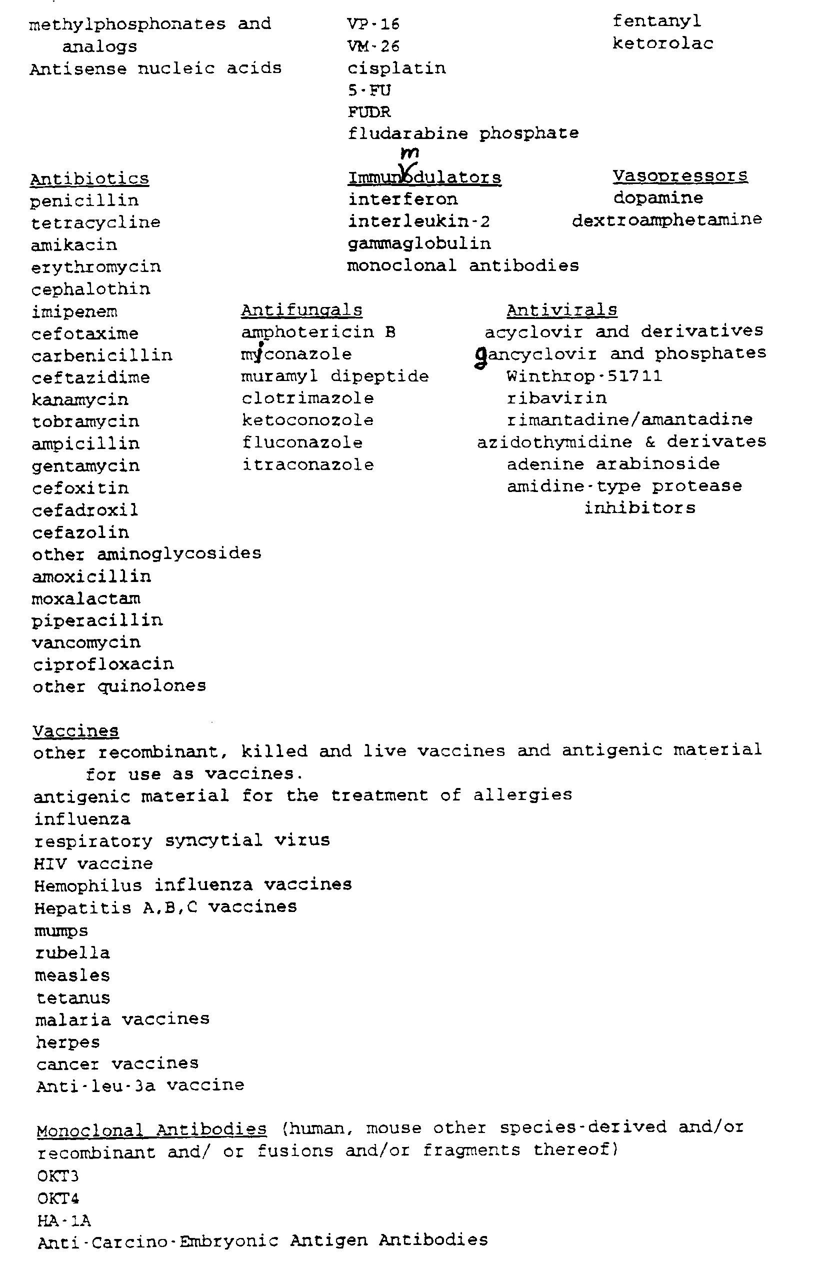

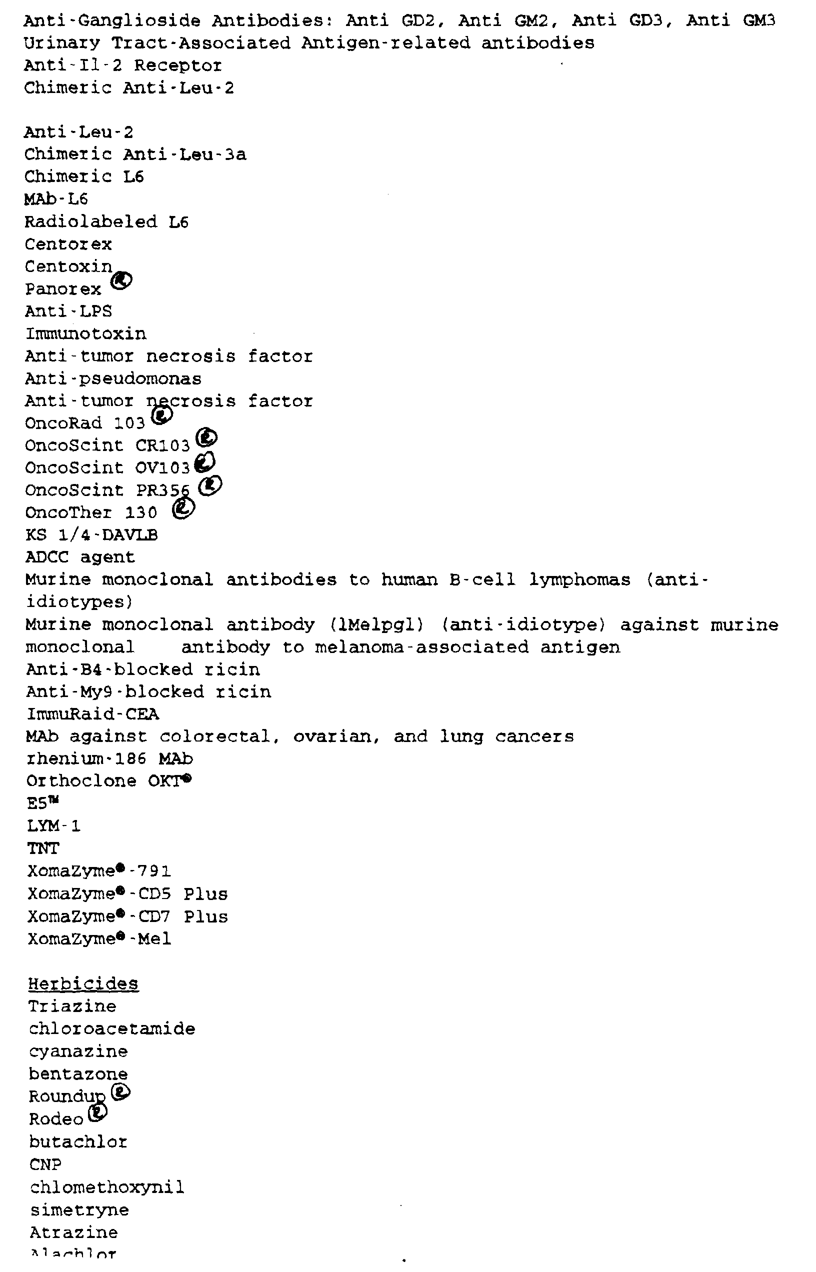

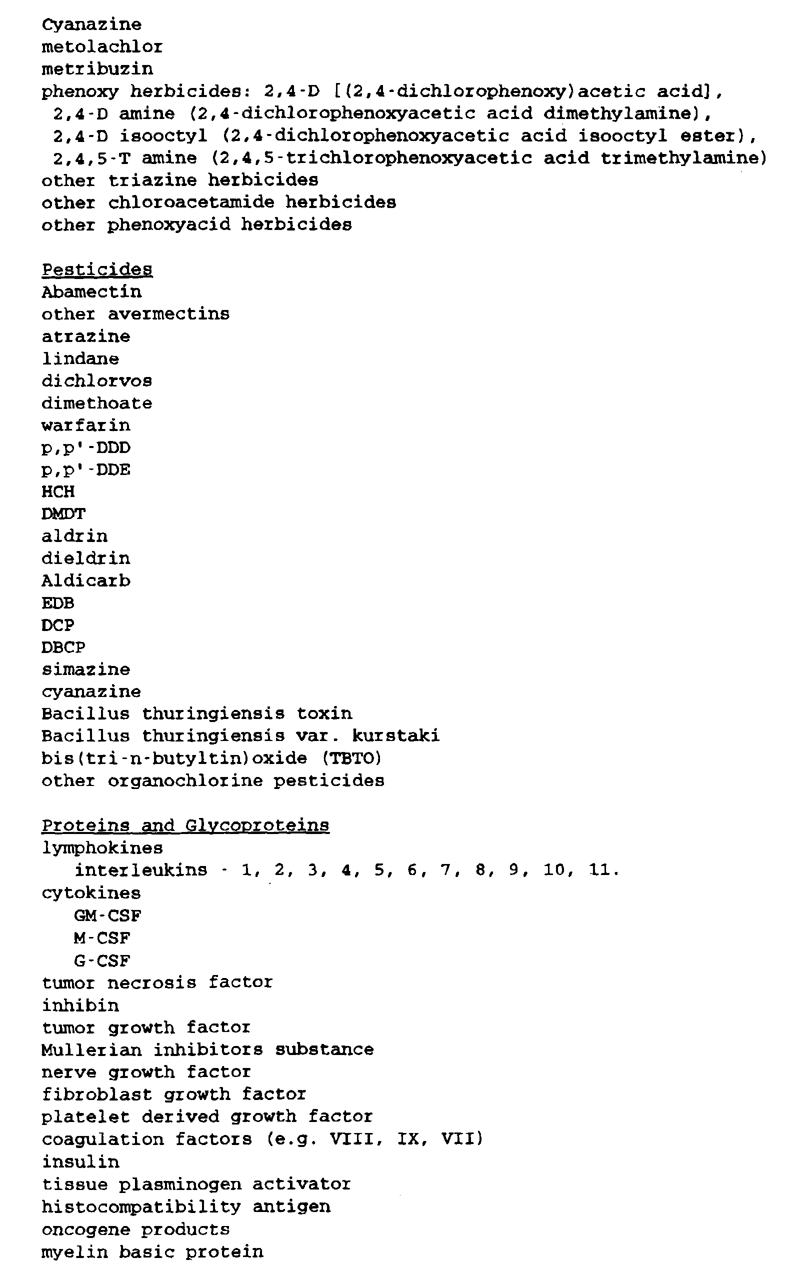

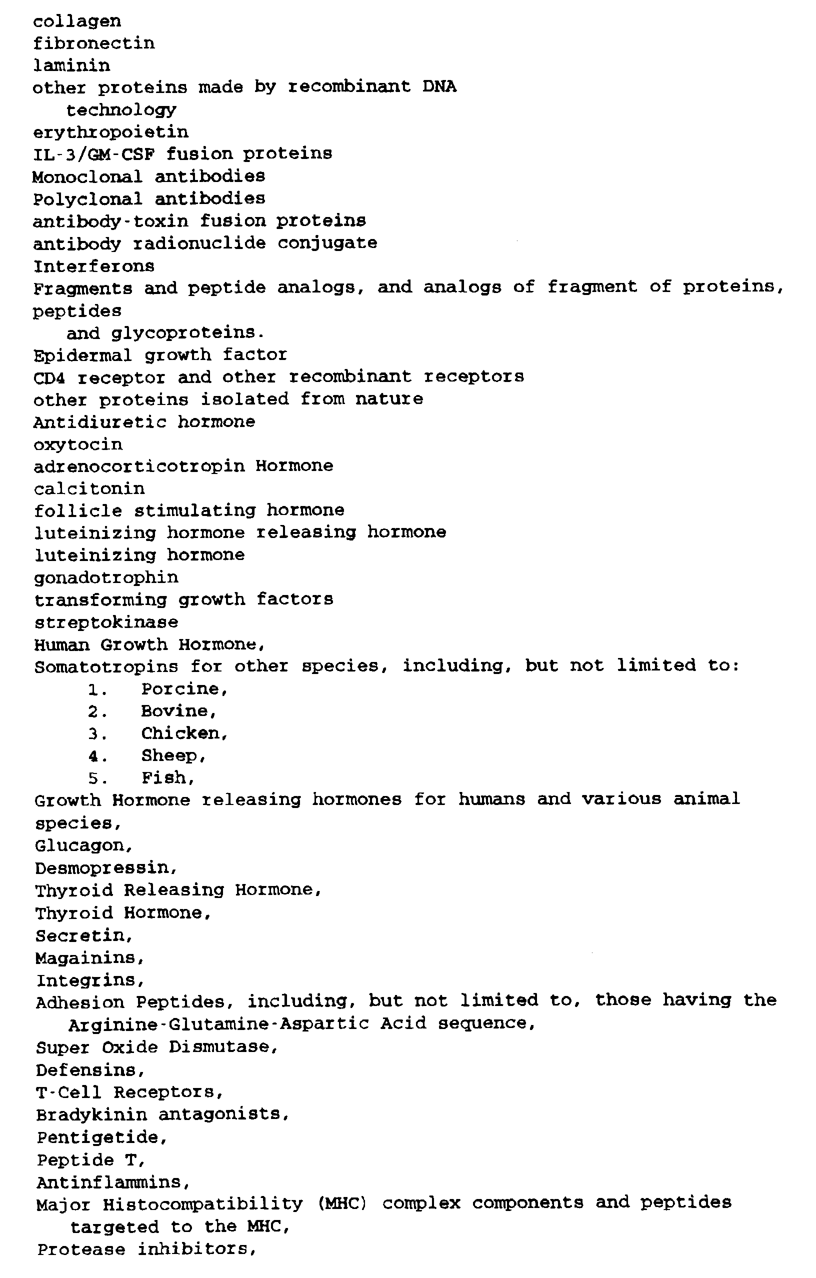

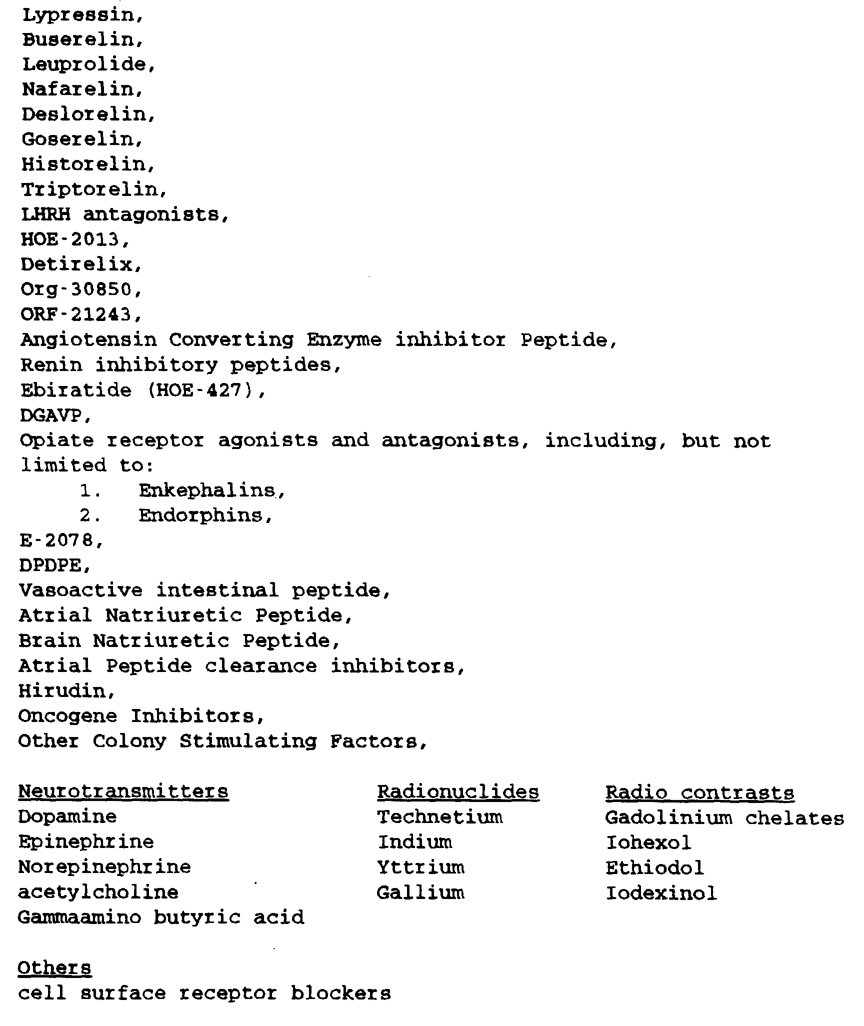

The term "therapeutically effective" as it pertains to the compositions of the invention means that a therapeutic agent is present in the aqueous phase within the vesicles at a concentration sufficient to achieve a particular medical effect for which the therapeutic agent is intended. Examples, without limitation, of desirable medical effects that can be attained are chemotherapy, antibiotic therapy, and regulation of metabolism. Exact dosages will vary depending upon such factors as the particular therapeutic agent and desirable medical effect, as well as patient factors such as age, sex, general condition, and the like. Those of skill in the art can readily take these factors into account and use them to establish effective therapeutic concentrations without resort to undue experimentation.

Generally, however, the dosage range appropriate for human use includes the range of 0.1-6000 mg/m² of body surface area. For some applications, such as subcutaneous administration, the dose required may be quite small, but for other applications, such as intraperitoneal administration, the dose desired to be used may be very large. While doses outside the foregoing dose range may be given, this range encompasses the breadth of use for practically all the biologically active substances.

The synthetic membrane vesicles may be administered for therapeutic applications by any desired route; for example, intramuscular, intrathecal, intraperitoneal, subcutaneous, intravenous, intralymphatic, oral and submucosal, under many different kinds of epithelia including the bronchialar epithelia, the gastrointestinal epithelia, the urogenital epithelia, and various mucous membranes of the body.

In addition the synthetic membrane vesicles of the invention can be used to encapsulate compounds useful in agricultural applications, such as fertilizers, pesticides, and the like. For use in agriculture, the synthetic membrane vesicles can be sprayed or spread onto an area of soil where plants will grow and the agriculturally effective compound contained in the vesicles will be released by contact with rain and irrigation waters. Alternatively the slow-releasing vesicles can be mixed into irrigation waters to be applied to plants and crops. One skilled in the art will be able to select an effective amount of the compound useful in agricultural applications to accomplish the particular goal desired, such as the killing of pests, the nurture of plants, etc.

The synthetic membrane vesicles may be modified in order to impart organ or cell target specificity, for instance by incorporating them into a targeted delivery system. Such modifications may be particularly relevant for using the synthetic membrane vesicles of the invention to administer drugs that are highly toxic or capable of inducing severe side effects, such as taxol.

The targeting of the synthetic membrane vesicles is classified based on anatomical and mechanistic factors. In anatomical targeting, the synthetic membrane vesicle is targeted to a specific body location, for example, organ-specific, cell-specific, and organelle-specific targeting. Mechanistic targeting can be distinguished based upon whether it is passive or active. Passive targeting utilizes the natural tendency of the synthetic membrane vesicles of the invention to distribute to cells of the reticulo-endothelial system (RES) in organs which contain sinusoidal capillaries. In active targeting, on the other hand, the synthetic membrane vesicle is incorporated into a targeted delivery system by coupling it to a specific ligand, such as a monoclonal antibody, sugar, glycolipid, or protein, or by changing the composition or size of the synthetic membrane vesicles in order to achieve targeting to organs and cell types other than the naturally occurring sites of localization (see, for example, *Remington's Pharmaceutical Sciences*, Gannaro, A.R., ed., Mack Publishing, 18 Edition, pp. 1691-1693, 1990)

In general, the compounds to be bound to the surface of the synthetic membrane vesicles will be ligands and receptors that allow the dispersion system to actively "home in" on the desired tissue. A ligand may be any compound of interest that will specifically bind to another compound, referred to as a receptor, such that the ligand and receptor form a homologous pair.

The surface of the targeted delivery system can be modified in a variety of ways. For instance, lipid groups can be incorporated into the lipid bilayer of the synthetic membrane vesicles in order to maintain the targeting ligand in stable association with the lipid bilayer. Various linking groups can be used for joining the lipid chains to the targeting ligand (Mannino, *et al., Bio Techniques,* 6(7):682, 1988). The compounds bound to the surface of the synthetic membrane vesicles may vary from small haptens of from about 125-200 M.W. Daltons to much larger antigens with molecular weights of at least about 6000 Daltons, but generally of less than 1 million Daltons. Proteinaceous ligands and receptors are of particular interest.

In general, the surface membrane proteins that bind to specific effector molecules are referred to as receptors. In the present invention, the preferred receptors are antibodies. These antibodies may be monoclonal or polyclonal and may be fragments thereof such as Fab F(ab')₂, and Fᵥ, which are capable of binding to an epitopic determinant. Techniques for binding of proteins, such as antibodies, to synthetic membrane vesicles are well known (see, for example, U.S. 4,806,466 and U.S. 4,857,735,).

Antibodies can be used to target the synthetic membrane vesicles to specific cell-surface ligands. For example, certain antigens expressed specifically on tumor cells, referred to as tumor-associated antigens (TAAs) may be exploited for the purpose of targeting antibody-containing synthetic membrane vesicles directly to malignant tumors. Since the composition incorporated into the synthetic membrane vesicles may be indiscriminate with respect to cell type in its action, targeted synthetic membrane vesicles offers a significant improvement over randomly injecting non-specific synthetic membrane vesicles. A number of procedures can be used to covalently attach either polyclonal or monoclonal antibodies to a bilayer of the synthetic membrane vesicles. Antibody-targeted synthetic membrane vesicles can include monoclonal or polyclonal antibodies or fragments thereof such as Fab, or F(ab')₂, as long as they bind efficiently to the antigenic epitope on the target cells. Synthetic membrane vesicles may also be targeted to cells expressing receptors for hormones or other serum factors (Malone, *et al., Proc. Nat'l. Acad. Sci, USA,* 86:6077, 1989; Gregoriadis, Immunology Today, 11(3):89, 1990;).

### EXAMPLE 1

Step 1) In a clean glass cylinder (2.5 cm inner diameter X 10.0 cm height), 5 ml of a solution containing 46.5 µmoles of dioleoyl phosphatidylcholine, 10.5 µmoles of dipalmitoyl phosphatidylglycerol, 75 µmoles of cholesterol, 9.0 µmoles of triolein in chloroform were placed (the lipid phase).
Step 2) Five ml of aqueous phase, cytarabine (20 mg/ml) dissolved in 0.136 N perchloric acid, a release-rate modifying agent, is added into the above glass cylinder containing lipid phase. The osmolarity of the aqueous solution is about 274 ± 20 mOs/kg. For the other release-rate modifying agents namely, nitric acid, formic acid, sulfuric acid, phosphoric acid, acetic acid, trichloroacetic acid, and trifluoroacetic acid, 20 mg/ml solutions of cytarabine were prepared with these agents to yield aqueous solutions that are nearly isotonic with respect to the final storage medium, namely normal saline (0.9% sodium chloride).
Step 3) For making the water-in-oil emulsion, a homogenizer (AutoHomoMixer, Model M, Tokushu Kika, Osaka, Japan) was used by mixing for 8 minutes at a speed of 9000 rpm.
Step 4) For making the chloroform spherules suspended in water, 20 ml of a solution containing 4 percent dextrose and 40 mM lysine was layered on top of the water-in-oil emulsion, and then mixed for 60 seconds at a speed of 4000 rpm to form the chloroform spherules.
Step 5) The chloroform spherule suspension in the glass cylinder was poured into the bottom of a 1000 ml Erlenmeyer flask containing 30 ml of water, glucose (3.5 g/100 ml), and free-base lysine (40 mM). A stream of nitrogen gas at 7 l/minute was flushed through the flask to slowly evaporate chloroform over 20 minutes at 37°C. 60 ml of normal saline (0.9% sodium chloride) was added to the flask. The synthetic membrane vesicles were then isolated by centrifugation at 600 X g for 10 minutes. The supernatant was decanted, and the pellet was resuspended in 50 ml of normal saline. The pellet was resuspended in saline to yield a final concentration of 10 mg cytarabine per ml of suspension.

The average length-weighted mean diameter of the resulting synthetic membrane vesicles particles was in the range from 12-16 µm. Percentage of capture of cytarabine is given in TABLE 2. The use of different release-modifying agents had marked influence on the rate of cytarabine release from the synthetic membrane vesicles incubated in human plasma. The percent of cytarabine retained in the synthetic membrane vesicles after incubation at 37°C in human plasma for the different acids is plotted as a function of time of incubation in Figure 1. The half-life of drug release, calculated assuming a single-exponential model for the data shown in Figure 1, is given in TABLE 2. The data in TABLE 2 are the mean and standard deviation from three experiments.

**TABLE 2**

| **Acid** | **Percent Capture of Cytarabine** | **Half-Life in Days for Release of Cytarabine** | **Symbol in Figure 1** |
|---|---|---|---|
| Hydrochloric Acid | 49 ± 5 | 65.7 ± 4.4 | ◆ |
| Perchloric Acid | 45 ± 5 | 37.2 ± 8.0 | ▼ |
| Nitric Acid | 44 ± 3 | 54.5 ± 5.7 | ■ |
| Phosphoric Acid | 72 ± 1 | 6.5 ± 0.2 | ▲ |
| Formic Acid | 37 ± 2 | 5.6 ± 0.2 | ◇ |
| Trichloroacetic Acid | 29 ± 1 | 5.5 ± 0.6 | ∇ |
| Acetic Acid | 30 ± 2 | 4.8 ± 0.5 | □ |
| Trifluoroacetic Acid | 35 ± 1 | 3.4 ± 0.4 | △ |
| Sulfuric Acid | 57 ± 4 | 1.6 ± 0.5 | ○ |

It was surprising and unexpected that the nature of the acid had a profound effect on the release rates of cytarabine in human plasma. Use of monoprotic inorganic acids, namely, hydrochloric acid, nitric acid, and perchloric acid, resulted in the slowest release rate for cytarabine. Diprotic and triprotic acids, i.e., sulfuric acid and phosphoric acid, resulted in fast release rates. The organic acids, formic acid, acetic acid, trifluoroacetic acid and trichloroacetic acid, also resulted in fast release rates.

Thus, the present disclosure provides "depot" preparations of wide application and uses in which biologically active substances are encapsulated in relatively large amounts, provide prolonged exposure or delivery at therapeutic concentrations of these substances for optimal results, and the release rate of the substance is controlled by varying the nature of the acid used in the formulation.

The present invention, therefore, is well suited and adapted to attain the ends and objects and has the advantages and features mentioned as well as others inherent therein.

## Claims

1. A composition comprising a synthetic membrane vesicle comprising lipid bilayer membranes enclosing multiple non-concentric aqueous chambers containing one or more biologically active substances encapsulated therein and one or more non-hydrohalide release-rate modifying agents.

2. The composition of claim 1, wherein the release-rate modifying agents are selected from the group consisting of nitric acid, perchloric acid, formic acid, sulfuric acid, phosphoric acid, acetic acid, trichloroacetic acid, and trifluoroacetic acid, and salts or combinations thereof.

3. The composition of claim 1, wherein the release-rate modifying agent is a monoprotic inorganic acid.

4. The composition of claim 2, wherein the acids are neutralized with a proton acceptor.

5. The composition of claim 1, wherein the biologically active substance is a drug.

6. The composition of claim 1, wherein the biologically active substances are selected from the group consisting of antibiotics, vaccines, antivirals, antifungals, anti-tumor drugs, proteins and glycoproteins.

7. A composition of claim 6, wherein the anti-tumor drug is cytarabine.

8. A composition of claim 1, wherein the biologically active substances are selected from the group consisting of herbicides and pesticides.

9. A targeted delivery system comprising a composition of claim 1 with a targeting ligand attached thereto.

10. A targeted delivery system of claim 9, wherein the targeting ligand is an antibody or fragment thereof.

11. A targeted delivery system of claim 10, wherein the antibody is a monoclonal antibody.

12. A targeted delivery system of claim 9, wherein lipid groups are incorporated into the lipid bilayer of the synthetic membrane vesicle.

13. The composition of claim 1, wherein the synthetic membrane vesicle is anatomically targeted.

14. The composition of claim 1, wherein the synthetic membrane vesicle is mechanistically targeted.

15. The composition of claim 1, wherein the synthetic membrane vesicle is passively targeted.

16. The composition of claim 1, wherein the synthetic membrane vesicle is actively targeted.

17. The composition of claim 16, wherein the synthetic membrane vesicle is actively targeted by coupling with a moiety selected from the group consisting of a sugar, a glycolipid, and a protein.

18. A synthetic membrane vesicle of claim 1 produced by the method comprising:
(a) forming a water-in-oil emulsion from two immiscible components containing at least one organic solvent, water, at least one biologically active substance, and at least one non-hydrohalide release-rate modifying agent;
(b) dispersing the said water-in-oil emulsion into an aqueous component to form solvent spherules; and
(c) removing the organic solvent from the solvent spherules to form the synthetic membrane vesicle.

19. A process for producing synthetic membrane vesicles comprising the steps of:
(a) forming a water-in-oil emulsion from two immiscible components containing at least one organic solvent, water, at least one biologically active substance, and at least one non-hydrohalide release-rate modifying agent;
(b) dispersing the water-in-oil emulsion into an aqueous component to form solvent spherules; and
(c) removing the organic solvent from the solvent spherules to form the synthetic membrane vesicles containing aqueous droplets with the biologically active substance and the release rate modifying agent dissolved therein..

20. The process of claim 19, wherein the concentration of the non-hydrohalide release-rate modifying agent is present in the range of about 0.1 mM to about 0.5 M.

21. The process of claim 19, wherein an acid neutralizing agent in a concentration of from about 0.1 mM to about 0.5 M is added during step (b).

22. The process of claim 19, wherein the organic solvent has a dissolved lipid component containing at least one amphipathic lipid with a net negative charge and at least one neutral lipid.

23. The process according to claim 22, wherein the lipid component is selected from the group consisting of a phospholipid and an admixture of phospholipids.

24. The process according to claim 23, wherein the phospholipids are selected from the group consisting of phosphatidylcholine, cardiolipin, phosphatidylethanolamine, sphingomyelin, lysophosphatidylcholine, phosphatidylserine, phosphatidylinositol, phosphatidylglycerol, and phosphatidic acid.

25. The process according to claim 24, wherein at least one of the phospholipids has at least one net negative charge.

26. The process according to claim 24, wherein the phospholipid is provided in admixture with cholesterol.

27. The process according to claim 24, wherein the phospholipid is provided in admixture with stearylamine.

28. The process according to claim 22, wherein a lipophilic biologically active material is provided in admixture with the lipid component.

29. The process according to claim 22, wherein the neutral lipid is selected from the group consisting of triolein, trioctanoin, vegetable oil, lard, beef fat, tocopherol, and combinations thereof.

30. The process according to claim 19, wherein the organic solvent is selected from the group consisting of ethers, hydrocarbons, halogenated hydrocarbons, halogenated ethers, esters, and combinations thereof.

31. The process according to claim 19, wherein the biologically active material is hydrophilic.

32. The process according to claim 19, wherein the emulsion is formed using a method selected from the group consisting of mechanical agitation, ultrasonic energy, and nozzle atomization.

33. The process according to claim 32, wherein the average size of the synthetic membrane vesicles and number of the aqueous chambers therewithin are determined by the type, intensity, and duration of the emulsification method selected.

34. The process according to claim 19, wherein the release rate modifying agent is a monoprotic inorganic acid, and aqueous component contains at least one neutralizing agent.

35. The process according to claim 34, wherein the neutralizing agent is selected from the group consisting of free-base lysine, free-base histidine, and a combination thereof.

36. The process according to claim 34, wherein the aqueous component is an aqueous solution containing solutes selected from the group consisting of carbohydrates and amino acids.

37. The process according to claim 34, wherein the aqueous component is an aqueous solution containing solutes selected from the group consisting of glucose, sucrose, lactose, free-base lysine, free-base histidine, and combinations thereof.

38. The process according to claim 19, wherein the solvent spherules are formed using a method selected from the group consisting of mechanical agitation, ultrasonic energy, nozzle atomization, and combinations thereof.

39. The process according to claim 38, wherein the average size or the synthetic membrane vesicle is determined by the type, intensity, and duration of the energy used.

40. The process according to claim 19, wherein the organic solvent is removed by passing gas over the aqueous component.

41. The process of Claim 19, wherein the biologically active substance is selected from the group consisting of antiasthmas, cardiac glycosides, antihypertensives, antiparasitics, nucleic acids and analogs, antibiotics, vaccines, antiarrhythmics, antianginas, hormones, antidiabetics, antineoplastics, immunomodulators, antifungals, tranquilizers, steroids, sedatives and analgesics, vasopressors, antivirals, monoclonal antibodies, herbicides, pesticides, proteins and glycoproteins, neurotransmitters, radionuclides, radio contrasts, and combinations thereof.

42. The synthetic membrane vesicle prepared in accordance with claim 32 or 33 wherein, the biologically active substance is selected from the group consisting of antiasthmas, cardiac glycosides, antihypertensives, antiparasitics, nucleic acids and analogs, antibiotics, vaccines, antiarrhythmics, antianginas, hormones, antidiabetics, antineoplastics, immunomodulators, antifungals, tranquilizers, steroids, sedatives and analgesics, vasopressors, antivirals, monoclonal antibodies, herbicides, pesticides, proteins and glycoproteins, neurotransmitters, radionuclides, radio contrasts, and combinations thereof.

43. Use of a biologically active compound encapsulated in a synthetic mebrane vesicle comprising lipid bilayer membranes enclosing multiple non concentric aqueous chambers in the presence of a non-hydrohalide release-rate modifying agent for the preparation of a pharmaceutical composition.

44. Use of a synthetic membrane vesicle of claims 1, 2, 3, 4, 5, 6, 7, 8, 9 or 10 for the preparation of a pharmaceutical composition.

45. The method of claim 19, wherein the biologically active substance is selected from the group consisting of herbicides and pesticides.

46. A synthetic membrane vesicle of claim 1 produced by a method comprising:
a) forming a water-in-oil emulsion from two immiscible components, the two immiscible components being
1) a lipid component comprising at least one volatile organic solvent, at least one amphipathic lipid, and at least one neutral lipid lacking a head group; and
2) a first aqueous component;
said water-in-oil emulsion further comprising at least one non-hydrohalide acid and at least one biologically active substance, said non-hydrohalide acid and said biologically active substance being independently incorporated into either the lipid component, the first aqueous component, or both;
b) mixing the water-in-oil emulsion in a) with a second aqueous component to form solvent spherules; and
c) removing the organic solvent from the solvent spherules to form multivesicular liposomes;
wherein the non-hydrohalide acid concentration in the water-in-oil emulsion is selected to provide controlled release of the biologically active substance of the liposomes

## Patentansprüche

1. Zusammensetzung, die ein Vesikel mit synthetischer Membran enthält, welche lipide zweischichtige Membranen aufweist, die mehrere, nicht konzentrische wasserhaltige Kammern einschließen, die eine oder mehrere darin eingeschlossene biologisch wirksame Substanzen und eines oder mehrere hydrohalogenide, die Freisetzungsgeschwindigkeit modifizierende Mittel enthalten.

2. Zusammensetzung nach Anspruch 1, wobei die die Freisetzungsgeschwindigkeit modifizierenden Mittel ausgewählt sind aus der Gruppe bestehend aus Salpetersäure, Perchlorsäure, Ameisensäure, Schwefelsäure, Phosphorsäure, Essigsäure, Trichloressigsäure und Trifluoressigsäure, sowie Salzen und Kombinationen daraus.

3. Zusammensetzung nach Anspruch 1, wobei das die Freisetzungsgeschwindigkeit modifizierende Mittel eine monoprotische anorganische Säure ist.

4. Zusammensetzung nach Anspruch 2, wobei die Säuren mit einem Protonenakzeptor neutralisiert werden.

5. Zusammensetzung nach Anspruch 1, wobei die biologisch wirksame Substanz ein Medikament ist.

6. Zusammensetzung nach Anspruch 1, wobei die biologisch wirksamen Substanzen ausgewählt sind aus der Gruppe bestehend aus Antibiotika, Impfstoffen, Antivirusmitteln, Antipilzmitteln, Medikamenten zur Tumorbekämpfung, Proteinen und Glykoproteinen.

7. Zusammensetzung nach Anspruch 6, wobei das Medikament zur Tumorbekämpfung Cytarabin ist.

8. Zusammensetzung nach Anspruch 1, wobei die biologisch wirksamen Substanzen ausgewählt sind aus der Gruppe bestehend aus Herbiziden und Pestiziden.

9. System zur gezielten Abgabe, welches eine Zusammensetzung nach Anspruch 1 mit einem daran gebundenen Zielliganden aufweist.

10. System zur gezielten Abgabe nach Anspruch 9, wobei der Zielligand ein Antikörper oder ein Bruchstück davon ist.

11. System zur gezielten Abgabe nach Anspruch 10, wobei der Antikörper ein monoklonaler Antikörper ist.

12. System zur gezielten Abgabe nach Anspruch 9, wobei Lipidgruppen in die Lipid-Doppelschicht des Vesikels mit synthetischer Membran eingebaut sind.

13. Zusammensetzung nach Anspruch 1, wobei das Vesikel mit synthetischer Membran anatomisch zielgesteuert ist.

14. Zusammensetzung nach Anspruch 1, wobei das Vesikel mit synthetischer Membran mechanistisch zielgesteuert ist.

15. Zusammensetzung nach Anspruch 1, wobei das Vesikel mit synthetischer Membran passiv zielgesteuert ist.

16. Zusammensetzung nach Anspruch 1, wobei das Vesikel mit synthetischer Membran aktiv zielgesteuert ist.

17. Zusammensetzung nach Anspruch 16, wobei das Vesikel mit synthetischer Membran aktiv zielgesteuert wird durch Verknüpfung mit einem Teil ausgewählt aus der Gruppe bestehend aus einem Zucker, einem Glykolipid und einem Protein.

18. Vesikel mit synthetischer Membran nach Anspruch 1, hergestellt durch ein Verfahren, das folgende Schritte aufweist:
(a) Bildung einer Wasser-in-Öl-Emulsion aus zwei unmischbare Bestandteilen, die mindestens ein organisches Lösemittel, Wasser, mindestens eine biologisch wirksame Substanz und mindestens ein nicht hydrohalogenides, die Freisetzungsgeschwindigkeit modifizierendes, Mittel enthält;
(b) Dispergieren der Wasser-in-Öl-Emulsion in einen wäßrigen Bestandteil, um Lösemittelkügelchen zu bilden;
(c) Entfernen des organischen Lösemittels aus den Lösemittelkügelchen, um das Vesikel mit synthetischer Membran zu bilden.

19. Verfahren zur Herstellung von Vesikeln mit synthetischer Membran, umfassend folgende Schritte:
(a) Bildung einer Wasser-in-Öl-Emulsion aus zwei unmischbaren Bestandteilen, die mindestens ein organisches Lösemittel, Wasser, mindestens eine biologisch wirksame Substanz und mindestens ein nicht hydrohalogenides, die Freisetzungsgeschwindigkeit modifizierendes, Mittel enthält;
(b) Dispergieren der Wasser-in-Öl-Emulsion in einen wäßrigen Bestandteil, um Lösemittelkügelchen zu bilden;
(c) (c) Entfernen des organischen Lösemittels aus den Lösemittelkügelchen, um die Vesikel mit synthetischer Membran zu bilden, die wäßrige Tröpfchen mit der biologisch wirksamen Substanz und, darin aufgelöst, das die Freisetzungsgeschwindigkeit modifizierende Mittel enthalten.

20. Verfahren nach Anspruch 19, wobei die Konzentration des nicht hydrohalogeniden, die Freisetzungsgeschwindigkeit modifizierenden Mittels im Bereich von etwa 0,1 mM bis etwa 0,5 M vorliegt.

21. Verfahren nach Anspruch 19, wobei ein säureneutralisierendes Mittel in einer Konzentration von etwa 0,1 mM bis etwa 0,5 M im Laufe von Schritt (b) hinzugefügt wird.

22. Verfahren nach Anspruch 19, wobei das organische Lösemittel einen gelösten Lipidbestandteil aufweist, der mindestens ein amphipatisches Lipid mit einer negativen Nettoladung und mindestens ein neutrales Lipid enthält.

23. Verfahren nach Anspruch 22, wobei der Lipidbestandteil ausgewählt ist aus der Gruppe bestehend aus einem Phospholipid und einer Beimengung von Phospholipiden.

24. Verfahren nach Anspruch 23, wobei die Phospholipide ausgewählt sind aus der Gruppe bestehend aus Phosphatidylcholin, Kardiolipin, Phosphatidylethanolamin, Sphingomyelin, Lysophosphatidylcholin, Phosphatidylserin, Phosphatidylinositol, Phosphatidylglycerin und Phosphatidsäure.

25. Verfahren nach Anspruch 24, wobei mindestens eines der Phospholipide mindestens eine negative Nettoladung aufweist.

26. Verfahren nach Anspruch 24, wobei das Phospholipid in Beimengung mit Cholesterol zur Verfügung gestellt ist.

27. Verfahren nach Anspruch 24, wobei das Phospholipid in Beimengung mit Stearylamin zur Verfügung gestellt ist.

28. Verfahren nach Anspruch 22, wobei ein lipophiles biologisch wirksames Material im Beimengung mit dem Lipidbestandteil zur Verfügung gestellt ist.

29. Verfahren nach Anspruch 22, wobei das neutrale Lipid ausgewählt ist aus der Gruppe bestehend aus Triolein, Trioktanoin, Pflanzenöl, Schweinefett, Rinderfett, Tokopherol und Kombinationen daraus. Verfahren nach Anspruch 19, wobei das organische Lösemittel ausgewählt ist aus der Gruppe bestehend aus Ethern, Kohlenwasserstoffen, halogenierten Kohlenwasserstoffen, halogenierten Ethern, Estern und Verbindungen daraus.

30. Verfahren nach Anspruch 19, wobei das biologisch wirksame Material hydrophil ist.

31. Verfahren nach Anspruch 19, wobei die Emulsion gebildet wird durch Verwendung eines Verfahrens ausgewählt aus der Gruppe bestehend aus mechanischem Rühren, Ultraschallenergie und Düsenversprühung.

32. Verfahren nach Anspruch 32, wobei die durchschnittliche Größe der Vesikel mit synthetischer Membran und die Zahl der wäßrigen Kammern darin durch die Art, Stärke und Dauer des ausgewählten Emulgierungsverfahrens bestimmt werden.

33. Verfahren nach Anspruch 19, wobei das die Freisetzungsgeschwindigkeit modifizierende Mittel eine monoprotische anorganische Säure ist, und der wäßrige Bestandteil mindestens ein Neutralisationsmittel enthält.

34. Verfahren nach Anspruch 34, wobei das Neutralisationsmittel ausgewählt ist aus der Gruppe bestehend aus als freie Base vorliegendem Lysin, als freie Base vorliegendem Histidin und einer Kombination daraus.

35. Verfahren nach Anspruch 34, wobei der wäßrige Bestandteil eine wäßrige Lösung ist, die gelöste Stoffe, ausgewählt aus der Gruppe bestehend aus Kohlenhydraten und Aminosäuren, enthält.

36. Verfahren nach Anspruch 34, wobei der wäßrige Bestandteil eine wäßrige Lösung ist, die gelöste Stoffe, ausgewählt aus der Gruppe bestehend aus Glucose, Saccharose, Lactose, als freie Base vorliegendem Lysin, als freie Base vorliegendem Histidin und Kombinationen daraus, enthält.

37. Verfahren nach Anspruch 19, wobei die Lösemittelkügelchen gebildet werden unter Verwendung eines Verfahrens ausgewählt aus der Gruppe bestehend aus mechanischem Rühren, Ultraschallenergie, Düsenversprühen und Kombinationen daraus.

38. Verfahren nach Anspruch 38, wobei die durchschnittliche Größe des Vesikels mit synthetischer Membran durch die Art, Stärke und Dauer der verwendeten Energie bestimmt wird.

39. Verfahren nach Anspruch 19, wobei das organische Lösemittel durch Durchleiten von Gas über den wäßrigen Bestandteil entfernt wird.

40. Verfahren von Anspruch 19, wobei die biologisch wirksame Substanz ausgewählt ist aus der Gruppe bestehend aus Asthmamitteln, Herzglykosiden, Antihypertonika, Antiparasitika, Nucleinsäuren und analogen Verbindungen, Antibiotika,

41. Impfstoffen, Arrhythmiemitteln, Anginamitteln, Hormonen, Antidiabetika, antineoplastischen Mitteln, Immunomodulatoren, Pilzmitteln, Beruhigungsmitteln, Steroiden, Sedativa und Analgetika, Kreislaufmitteln, Antivirusmitteln, monoklonalen Antikörpern, Herbiziden, Pestiziden, Proteinen und Glykoproteinen, Neurotransmittern, Radionukliden, Röntgenkontrastmitteln und Kombinationen daraus.

42. Vesikel mit synthetischer Membran, hergestellt entsprechend Anspruch 32 oder 33, wobei die biologisch wirksame Substanz ausgewählt ist aus der Gruppe bestehend aus Asthmamitteln, Herzglykosiden, Antihypertonika, Antiparasitika, Nucleinsäuren und analogen Verbindungen, Antibiotika, Impfstoffen, Arrhythmiemitteln, Anginamitteln, Hormonen, Antidiabetika, antineoplastischen Mitteln, Immunomodulatoren, Pilzmitteln, Beruhigungsmitteln, Steroiden, Sedativa und Analgetika, Kreislaufmitteln, Antivirusmitteln, monoklonalen Antikörpern, Herbiziden, Pestiziden, Proteinen und Glykoproteinen, Neurotransmittern, Radionukliden, Röntgenkontrastmitteln und Kombinationen daraus.

43. Verwendung einer biologisch wirksamen Verbindung, die in einem Vesikel mit synthetischer Membran eingeschlossen ist, die lipide zweischichtige Membranen, die mehrere nicht konzentrische wasserhaltige Kammern einschließen, aufweist, in Gegenwart eines nicht hydrohalogeniden, die Freisetzungsgeschwindigkeit modifizierenden, Mittels zur Herstellung einer pharmazeutischen Zusammensetzung.

44. Verwendung eines Vesikels mit synthetischer Membran von Anspruch 1, 2, 3, 4, 5, 6, 7, 8, 9 oder 10 zur Herstellung einer pharmazeutischen Zusammensetzung.

45. Verfahren nach Anspruch 19, wobei die biologisch wirksame Substanz ausgewählt ist aus der Gruppe bestehend aus Herbiziden und Pestiziden.

46. Vesikel mit synthetischer Membran nach Anspruch 1, hergestellt durch ein Verfahren umfassend:
a) Bildung einer Wasser-in-Öl-Emulsion aus zwei unmischbaren Bestandteilen, wobei die zwei unmischbaren Bestandteile folgende sind:
1) ein Lipidbestandteil mit mindestens einem flüchtigen organischen Lösemittel, mindestens einem amphipathischen Lipid und mindestens einem neutralen Lipid mit einer fehlenden Kopfgruppe; und
2) ein erster wäßriger Bestandteil;
wobei die Wasser-in-Öl-Emulsion weiters mindestens eine nicht hydrohalogenide Säure und mindestens eine biologisch wirksame Substanz aufweist, wobei die nicht hydrohalogenide Säure und die biologisch wirksame Substanz unabhängig entweder in den Lipidbestandteil oder in den ersten wäßrigen Bestandteil oder in beides eingebaut werden;
b) Mischen der Wasser-in-Öl-Emulsion in a) mit einem zweiten wäßrigen Bestandteil, um Lösemittelkügelchen zu bilden; und
c) Entfernen des organischen Lösemittels aus den Lösemittelkügelchen, um multivesikuläre Liposomen zu bilden;
wobei die Konzentration der nicht hydrohalogeniden Säure in der Wasser-in-Öl-Emulsion ausgewählt ist, um eine gesteuerte Freisetzung der biologisch wirksamen Substanz der Liposomen zu ermöglichen.

## Revendications

1. Composition comprenant une vésicule membraneuse synthétique comprenant des membranes lipidiques à deux couches enserrant de multiples chambres aqueuses non concentriques contenant une ou plusieurs substances biologiquement actives qui y sont encapsulées et un ou plusieurs agents de modification de la vitesse de libération non hydrohalogénurés.

2. Composition selon la revendication 1, dans laquelle les agents de modification de la vitesse de libération sont choisis dans le groupe constitué de l'acide nitrique, de l'acide perchlorique, de l'acide formique, de l'acide sulfurique, de l'acide phosphorique, de l'acide acétique, de l'acide trichloracétique et de l'acide trifluoroacétique et de leurs sels ou de leurs combinaisons.

3. Composition selon la revendication 1, dans laquelle l'agent de modification de la vitesse de libération est un acide inorganique monoprotique.

4. Composition selon la revendication 2, dans laquelle les acides sont neutralisés par un accepteur de proton.

5. Composition selon la revendication 1, dans laquelle la substance biologiquement active est un médicament.

6. Composition selon la revendication 1, dans laquelle les substances biologiquement actives sont choisies dans le groupe constitué des antibiotiques, des vaccins, des antiviraux, des antifongiques, des médicaments anti-tumoraux, des protéines et des glycoprotéines.

7. Composition selon la revendication 6, dans laquelle le médicament anti-tumoral est la cytarabine.

8. Composition selon la revendication 1, dans laquelle les substances biologiquement actives sont choisies dans le groupe constitué des herbicides et des pesticides.

9. Système de délivrance ciblée comprenant une composition selon la revendication 1, avec un ligand de ciblage qui lui est fixé.

10. Système de délivrance ciblée selon la revendication 9, dans lequel le ligand de ciblage est un anticorps ou un fragment de celui-ci.

11. Système de délivrance ciblée selon la revendication 10, dans lequel l'anticorps est un anticorps monoclonal.

12. Système de délivrance ciblée selon la revendication 9, dans lequel des groupes de lipides sont incorporés à la double couche lipidique de la vésicule membraneuse synthétique.

13. Composition selon la revendication 1, dans laquelle la vésicule membraneuse synthétique est ciblée par voie anatomique.

14. Composition selon la revendication 1, dans laquelle la vésicule membraneuse synthétique est ciblée par voie mécanique.

15. Composition selon la revendication 1, dans laquelle la vésicule membraneuse synthétique est ciblée passivement.

16. Composition selon la revendication 1, dans laquelle la vésicule membraneuse synthétique est ciblée activement.

17. Composition selon la revendication 16, dans laquelle la vésicule membraneuse synthétique est ciblée activement par couplage avec une partie choisie dans le groupe constitué d'un sucre, d'un glycolipide et d'une protéine.

18. Vésicule membraneuse synthétique selon la revendication 1 produite par le procédé comprenant les étapes consistant :
(a) à former une émulsion eau dans l'huile à partir de deux composants non miscibles contenant au moins un solvant organique, de l'eau, au moins une substance biologiquement active et au moins un agent de modification de la vitesse de libération non hydrohalogénuré;
(b) à disperser ladite émulsion eau dans l'huile dans un composant aqueux pour former des sphérules de solvant, et
(c) à éliminer le solvant organique des sphérules de solvant pour former la vésicule membraneuse synthétique.

19. Procédé de production de vésicules membraneuses synthétiques comprenant les étapes consistant :
(a) à former une émulsion eau dans l'huile à partir de deux composants non miscibles contenant au moins un solvant organique, de l'eau, au moins une substance biologiquement active et au moins un agent de modification de la vitesse de libération non hydrohalogénuré;
(b) à disperser l'émulsion eau dans l'huile dans un composant aqueux pour former des sphérules de solvant, et
(c) à éliminer le solvant organique des sphérules de solvant pour former les vésicules membraneuses synthétiques contenant des gouttelettes aqueuses, avec la substance biologiquement active et l'agent de modification de la vitesse de libération qui y sont dissous.

20. Procédé selon la revendication 19, dans lequel la concentration de l'agent de modification de la vitesse de libération non hydrohalogénuré se situe dans la plage d'environ 0,1 mmole à environ 0,5 mole.

21. Procédé selon la revendication 19, dans lequel un agent de neutralisation acide en concentration d'environ 0,1 mmole à environ 0,5 mole est ajouté au cours de l'étape (b).

22. Procédé selon la revendication 19, dans lequel le solvant organique a un composant lipidique dissous contenant au moins un lipide amphipathique avec une charge négative nette et au moins un lipide neutre.

23. Procédé selon la revendication 22, dans lequel le composant lipidique est choisi dans le groupe constitué d'un phospholipide et d'un mélange de phospholipides.

24. Procédé selon la revendication 23, dans lequel les phospholipides sont choisis dans le groupe constitué de la phosphatidylcholine, de la cardiolipine, de la phosphatidyléthanolamine, de la sphingomyéline, de la lysophosphatidylcholine, de la phosphatidylserine, du phosphatidylinositol, du phosphatidylglycérol et de l'acide phosphatidique.

25. Procédé selon la revendication 24, dans lequel au moins l'un des phospholipides a au moins une charge négative nette.

26. Procédé selon la revendication 24, dans lequel le phospholipide est fourni en mélange avec du cholestérol.

27. Procédé selon la revendication 24, dans lequel le phospholipide est fourni en mélange avec de la stéarylamine.

28. Procédé selon la revendication 22, dans lequel un matériau lipophile biologiquement actif est fourni en mélange avec le composant lipidique.

29. Procédé selon la revendication 22, dans lequel le lipide neutre est choisi dans le groupe constitué de la trioléine, de la trioctanoïne, de l'huile végétale, du saindoux, de la graisse de boeuf, du tocophérol et de combinaisons de ceux-ci.

30. Procédé selon la revendication 19, dans lequel le solvant organique est choisi dans le groupe constitué des éthers, des hydrocarbures, des hydrocarbures halogénés, des éthers halogénés, des esters et de combinaisons de ceux-ci.

31. Procédé selon la revendication 19, dans lequel le matériau biologiquement actif est hydrophile.

32. Procédé selon la revendication 19, dans lequel l'émulsion est formée en utilisant un procédé choisi dans le groupe constitué d'une agitation mécanique, d'une énergie à ultrasons et d'une atomisation par buse.

33. Procédé selon la revendication 32, dans lequel la taille moyenne des vésicules membraneuses synthétiques et le nombre de chambres aqueuses qui s'y trouvent sont déterminés par le type, l'intensité et la durée du procédé d'émulsionnement choisi.

34. Procédé selon la revendication 19, dans lequel l'agent de modification de la vitesse de libération est un acide inorganique monoprotique et le composant aqueux contient au moins un agent de neutralisation.

35. Procédé selon la revendication 34, dans lequel l'agent de neutralisation est choisi dans le groupe constitué de la lysine de base libre, de l'histidine de base libre et d'une combinaison de celles-ci.

36. Procédé selon la revendication 34, dans lequel le composant aqueux est une solution aqueuse qui contient des solutés choisis dans le groupe constitué d'hydrates de carbone et d'acides aminés.

37. Procédé selon la revendication 34, dans lequel le composant aqueux est une solution aqueuse qui contient des solutés choisis dans le groupe constitué du glucose, du saccharose, du lactose, de la lysine de base libre, de l'histidine de base libre et de combinaisons de ceux-ci.

38. Procédé selon la revendication 19, dans lequel les sphérules de solvant sont formées en utilisant un procédé choisi dans le groupe constitué d'une agitation mécanique, d'une énergie à ultrasons, d'une atomisation par buse et de combinaisons de celles-ci.

39. Procédé selon la revendication 38, dans lequel la taille moyenne de la vésicule membraneuse synthétique est déterminée par le type, l'intensité et la durée de l'énergie utilisée.

40. Procédé selon la revendication 19, dans lequel le solvant organique est éliminé en faisant passer du gaz sur le composant aqueux.

41. Procédé selon la revendication 19, dans lequel la substance biologiquement active est choisie dans le groupe constitué des anti-asthme, des glycosides cardiaques, des anti-hypertenseurs, des antiparasites, des acides nucléiques et analogues, des antibiotiques, des vaccins, des agents anti-arythmie, des agents anti-angine, des hormones, des antidiabétiques, des antinéoplastiques, des immunomodulateurs, des antifongiques, des tranquillisants, des stéroïdes, des sédatifs et des analgésiques, des vasopresseurs, des antiviraux, des anticorps monoclonaux, des herbicides, des pesticides, des protéines et des glycoprotéines, des neurotransmetteurs, des radionuclides, des radiocontrastes et de combinaisons de ceux-ci.

42. Vésicule membraneuse synthétique préparée selon la revendication 32 ou 33, dans laquelle la substance biologiquement active est choisie dans le groupe constitué des anti-asthme, des glycosides cardiaques, des anti-hypertenseurs, des antiparasites, des acides nucléiques et analogues, des antibiotiques, des vaccins, des anti-arythmie, des anti-angine, des hormones, des antidiabétiques, des antinéoplastiques, des immunomodulateurs, des antifongiques, des tranquillisants, des stéroïdes, des sédatifs et des analgésiques, des vasopresseurs, des antiviraux, des anticorps monoclonaux, des herbicides, des pesticides, des protéines et des glycoprotéines, des neurotransmetteurs, des radionuclides, des radiocontrastes et de combinaisons de ceux-ci.

43. Utilisation d'un composé biologiquement actif encapsulé dans une vésicule membraneuse synthétique comprenant des membranes lipidiques à deux couches renfermant de multiples chambres aqueuses non concentriques en présence d'un agent de modification de la vitesse de libération non hydrohalogénuré pour la préparation d'une composition pharmaceutique.

44. Utilisation d'une vésicule membraneuse synthétique selon les revendications 1, 2, 3, 4, 5, 6, 7, 8, 9 ou 10 pour la préparation d'une composition pharmaceutique.

45. Procédé selon la revendication 19, dans lequel la substance biologiquement active est choisie dans le groupe constitué des herbicides et des pesticides.

46. Vésicule membraneuse synthétique selon la revendication 1, produite par un procédé comprenant les étapes consistant :
(a) à former une émulsion eau dans l'huile à partir de deux composants non miscibles, les deux composants non miscibles étant :
1) un composant lipidique comprenant au moins un solvant organique volatil, au moins un lipide amphipathique et au moins un lipide neutre sans groupe de tête, et
2)un premier composant aqueux;
ladite émulsion eau dans l'huile comprenant en outre au moins un acide non hydrohalogénuré et au moins une substance biologiquement active, ledit acide non hydrohalogénuré et ladite substance biologiquement active étant incorporés indépendamment soit au composant lipidique, au premier composant aqueux soit aux deux,
(b) à mélanger l'émulsion eau dans l'huile de a) à un second composant aqueux pour former des sphérules de solvant, et
(c) à éliminer le solvant organique des sphérules de solvant pour former des liposomes multivésiculaires,
dans laquelle la concentration en acide non hydrohalogénuré dans l'émulsion eau dans l'huile est choisie pour assurer une libération contrôlée de la substance biologiquement active des liposomes.
